(19) Europäisches Patentamt
European Patent Office
Office européen des brevets

(11) **EP 4 295 754 A1**

(12) **EUROPEAN PATENT APPLICATION**

(43) Date of publication:
**27.12.2023 Bulletin 2023/52**

(21) Application number: **23180353.7**

(22) Date of filing: **20.06.2023**

(51) International Patent Classification (IPC):
**A61B 5/00** $^{(2006.01)}$ **A61B 5/024** $^{(2006.01)}$
**A61B 5/332** $^{(2021.01)}$ **A61B 5/346** $^{(2021.01)}$

(52) Cooperative Patent Classification (CPC):
**A61B 5/346; A61B 5/0006; A61B 5/02438;
A61B 5/332;** G16H 50/20

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **20.06.2022 TW 111122919**

(71) Applicant: **Chen, Kang-Ying
Taoyuan City 326022 (TW)**

(72) Inventor: **Chen, Kang-Ying
Taoyuan City 326022 (TW)**

(74) Representative: **Lang, Christian
LangPatent Anwaltskanzlei IP Law Firm
Ingolstädter Straße 5
80807 München (DE)**

(54) **METHOD FOR TRANSFORMING INDEX VALUE BASED ON ECG SIGNAL AND SYSTEM THEREOF**

(57)    A method and a system for transforming electrocardiogram (ECG) signals into an index value are provided. The system includes a sensing device (10) wirelessly connected with an electronic device (20) and used for sensing an electrocardiogram (ECG) signal. The ECG signal is uploaded to a server (30) through the electronic device (20). After the ECG signal processed by decomposition, feature extraction, and geometric computation in the server (30), a corresponding index value is generated and visually represented for allowing users to catch their heart status directly.

Fig. 2

**Description**

FIELD OF THE INVENTION

The present application is an operation method and a system thereof, especially to a method for transforming electrocardiogram (ECG) signals into an index value and a system thereof.

BACKGROUND OF THE INVENTION

According to world health statistics 2018 published by World Health Organization, an estimated 1,765 million of people died from ischemic heart disease and stroke, representing 31% of all global deaths (5.69 million) in 2016. Among ten major causes of death in Taiwan, half of the causes are related to cardiovascular diseases (CVD). The mortality rate in patients with cardiovascular disease is about 10%. Once in combination with cerebrovascular disease and complications of diabetes, the CVD mortality rate is up to 25%. Thus, specific tests for CVD is necessary for diagnosis of CVD. As to the electrocardiogram test, it has obvious difference before and after an external stimulate.

Generally, cardiovascular examinations are divided into two types, non-invasive type and invasive type. The most common and convenient one used now is non-invasive type such as exercise treadmill test which allows subjects to walk on a treadmill for increasing heart oxygen consumption and workload of the body. Under supervision of medical staff, the intensity of the exercise can be adjusted according to test status. Thus, the subject's heart changes along with the exercise status and so does the electrocardiography. Therefore, whether the subject has corresponding cardiovascular diseases can be inferred.

The above exercise ECG is a kind of stress test, using a non-invasive method to evaluate the degree of coronary artery diseases (CAD). During the exercise test, the workload is gradually increased so that heart rate and systolic blood pressure of the subject also rise safely. The double product which is the product of the heart rate and systolic blood pressure is an index of myocardial oxygen consumption. For example, the ECG of patients with CAD may be normal while they are at rest. But the amount of oxygen required is increased and is larger than the amount of oxygen supplied during exercise so that patients with no symptoms of CAD may have changes in their ECG signals which reflect changes in coronary arteries.

An electrocardiogram is a graph of voltage versus time of the electrical activity of the heart while the voltage is captured and recorded by placing electrode on the skin. Thus, professional staff can detect heart conditions by measurement devices. People need to go to medical facilities such as regional hospitals and make an appointment in order to see doctors and get the ECG test which is an assistant measurement in healthcare system.

Thus, people rarely seek medical attention and the related test actively as soon as possible.

However, subjects who are patients with heart diseases may be unable to complete all steps of a standard ECG test. Interpretation of ECG recordings is not easy for the general public and thus people need to go to medical institutions or centers for ECG tests and medical personnel interpret the ECG result and tell the subjects their cardiovascular status represented by the ECG.

There are various types of consumer products available on the market such as wearable devices provide users a simple way to measure their heart rate. Yet most of measurement are associated with recording or focused on heart-rate related analysis such as cardiac arrhythmia, dysautonomia, artrial fibrillation, etc. Thus, tests related to health status of the heart provided by the wearable device are unable to achieve the same effect as those carried out in hospitals. That means it is difficult to develop a single-lead ECG test provided by the wearable device, which must be convenient to carry out and able to provide easy-to-read test results at the same time.

Thus, there is room for improvement and there is a need to provide a method for transforming electrocardiogram (ECG) signals into an index value and a system thereof, in which a ECG signal is detected by a sensing device and then the ECG signal is sent to a server and processed by decomposition, feature extraction, and geometric computation to get a corresponding index value. Thereby users can catch their health status directly by the index value.

SUMMARY OF THE INVENTION

Therefore, it is a primary object of the present application to provide a method for transforming electrocardiogram (ECG) signals into an index value and a system thereof, in which ECG signals uploaded to a server by an electronic device are processed by decomposition MD, feature extraction FE, and geometric computation CC to get a corresponding index value. Thus, users can catch their health status according to the index value.

In order to achieve the above object, a method for transforming electrocardiogram (ECG) signals into an index value according to the present application is applied to a sensing device wirelessly connected with an electronic device and used for detecting a first ECG signal. First the first ECG signal is uploaded from the sensing device to a server through the electronic device. The first ECG signal is decomposed to generate a plurality of first components by the server. Then the server performs feature extraction according to the first components to generate a plurality of first feature values. Next the server carries out geometric computation based on the first feature values to generate a first index value. Thus, the present method for transforming electrocardiogram (ECG) signals into an index value gets the index value after a plurality of operation processes. The index value is visually represented for allowing users to catch their health status directly.

Preferably, in the step of performing geometric computation to generate a first index value according to the first feature values by the server, the server is set with a plurality of threshold values corresponding to the first index value. Thereby the server generates a first status message according to the threshold values and the first index value.

Preferably, after the step of performing geometric computation to generate a first index value according to the first feature values, the first index value is sent back to the electronic device for display on the electronic device.

Preferably, the electronic device is set with a plurality of threshold values corresponding to the first index value. Thus, the electronic device generates a first status message according to the threshold values and the first index value. Then the first index value and the first status message are shown on the electronic device.

Preferably, the sensing device further detects a second ECG signal and uploads the second ECG signal to the server through the electronic device. The method further includes the step of decomposing the second ECG signal to generate a plurality of second components by the server. Then feature extraction from the second components is performed to generate a plurality of second feature values. Next, geometric computation is carried out to generate a second index value according to the second feature values. That means each ECG signal can be converted into its corresponding index value.

Preferably, in the step of performing geometric computation to generate a second index value according to the second feature values by the server, the server is set with a plurality of threshold values corresponding to the second index value. The server further generates a second status message according to the threshold values and the second index value.

Preferably, the server sends the second index value back to the electronic device for displaying the second index value on the electronic device.

Preferably, the electronic device is set with a plurality of threshold values corresponding to the second index value. Thus, the electronic device generates a second status message according to the threshold values and the second index value. Then the second status message is shown on the electronic device.

In order to achieve the above object, a system for transforming electrocardiogram (ECG) signals into an index value includes a sensing device, an electronic device, and a server. The sensing device is wirelessly connected with the electronic device which is connected with the server. A first ECG signal is sensed by the sensing device and then uploaded to the server through the electronic device. The first ECG signal is decomposed to generate a plurality of first components by the server. Then the server performs feature extraction from the first components to generate a plurality of first feature values. Next the server carries out geometric computation based on the first feature values to generate a first index value.

Preferably, the server is set with a plurality of threshold values corresponding to the first index value. Thus, the server generates a first status message according to the threshold values and the first index value and then sends the first status message and the first index value back to the electronic device.

Preferably, the server sends the first index value back to the electronic device.

Preferably, the electronic device is set with a plurality of threshold values corresponding to the first index value. Thus, the electronic device generates a first status message according to the threshold values and the first index value. Then the first index value and the first status message are shown on the electronic device.

Preferably, the sensing device further senses a second ECG signal is sensed and then the second ECG signal is uploaded to the server by a first program. The second ECG signal is decomposed to generate a plurality of second components. Next, feature extraction from the second components is performed to generate a plurality of second feature values. Then geometric computation of the second feature values is performed to generate a second index value.

BRIEF DESCRIPTION OF THE DRAWINGS

The structure and the technical means adopted by the present application to achieve the above and other objects can be best understood by referring to the following detailed description of the preferred embodiments and the accompanying drawings, wherein:

Fig. 1 is a flow chart showing steps of a method for transforming electrocardiogram (ECG) signals into an index value of an embodiment according to the present application;

Fig. 2 is a schematic drawing showing transmission of ECG signals in a system for transforming electrocardiogram (ECG) signals into an index value of an embodiment according to the present application;

Fig. 3 is schematic drawing showing decomposition in a system of an embodiment according to the present application;

Fig. 4 is a schematic drawing showing feature extraction in a system of an embodiment according to the present application;

Fig. 5A is a schematic drawing showing generation of a first index value in a system of an embodiment according to the present application;

Fig. 5B is a schematic drawing showing a first index value being sent back in a system of an embodiment according to the present application;

Fig. 5C is a schematic drawing showing generation of a first status message by an electronic device in a system of another embodiment according to the present application;

Fig. 5D is a schematic drawing showing generation of a first status message by a wearable device in a system of a further embodiment according to the present application;

Fig. 6A is a schematic drawing showing generation of a first index value and a first status message in a system of another embodiment according to the present application;

Fig. 6B is a schematic drawing showing a first index value and a first status message being sent back in a system of another embodiment according to the present application;

Fig. 7 is a flow chart showing steps of another embodiment according to the present application;

Fig. 8A is a schematic drawing showing transmission in a system of another embodiment according to the present application;

Fig. 8B is a schematic drawing showing generation of a second index value in a system of another embodiment according to the present application;

Fig. 8C is a schematic drawing showing a second index value being sent back in a system of an embodiment according to the present application;

Fig. 9A is a schematic drawing showing generation of a second status message by an electronic device in a system of another embodiment according to the present application;

Fig. 9B is a schematic drawing showing generation of a second status message by a wearable device in a system of another embodiment according to the present application;

Fig. 10A is a schematic drawing showing generation of a second index value and a second status message in a system of another embodiment according to the present application;

Fig. 10B is a schematic drawing showing a second index value and a second status message being sent back in a system of another embodiment according to the present application;

Fig. 11 is a schematic drawing showing a system with a wearable device of another embodiment according to the present application;

Fig. 12 is a schematic drawing showing a system with a steering wheel of another embodiment according to the present application;

Fig. 13 is a schematic drawing showing a system with a treadmill of another embodiment according to the present application.

DETAILED DESCRIPTION OF THE PREFERRED EMBODIMENT

In order to catch technical content, purposes and functions of the present application more clearly and completely, please refer to the following detailed descriptions with the figures and reference signs.

In the following specifications and claims, certain terms are used to indicate specific components. Yet the same component may be indicated by different terms for people having ordinary skill in the art. Instead of the differences in terms, the components are differentiated by technical differences in the whole device. The word "includes" mentioned in the specification and the claims is open-ended, which means including but not limited to. Moreover, the word "couple" means direct and indirect connection means. For example, a first device is coupled to a second device. This means the first device can be connected to the second device directly or indirectly by other devices or connection means.

The conventional electrocardiogram test is a complicated task which needs to be performed at specific areas and ECG interpretation is not quite easy for people. In order to solve the problem, a method for transforming electrocardiogram (ECG) signals into an index value and a system thereof according to the present application upload ECG signals to a server. After decomposition, feature extraction, and geometric computation of the ECG signals by the server, a corresponding index value is obtained and visually represented for allowing people to catch their physical conditions directly.

Please refer to the following embodiments with detailed descriptions and the figures. The preferred embodiments are used to explain the present application, but not to limit the present application.

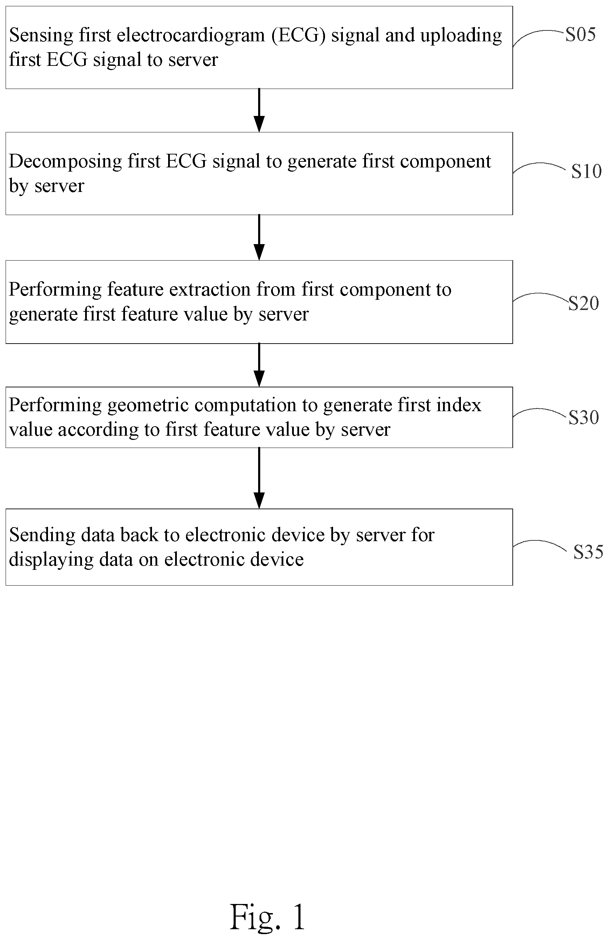

Refer to Fig. 1, a flow chart showing steps of a method for transforming electrocardiogram (ECG) signals into an index value according to the present application is provided. The method includes the following steps.

Step S05: Sensing first electrocardiogram (ECG) signal and uploading first ECG signal to server;

Step S10: Decomposing first ECG signal to generate first component by server;

Step S20: Performing feature extraction from first component to generate first feature value by server;

Step S30: Performing geometric computation to generate first index value according to first feature value by server;

Step S35: Sending data back to electronic device by server for displaying data on electronic device.

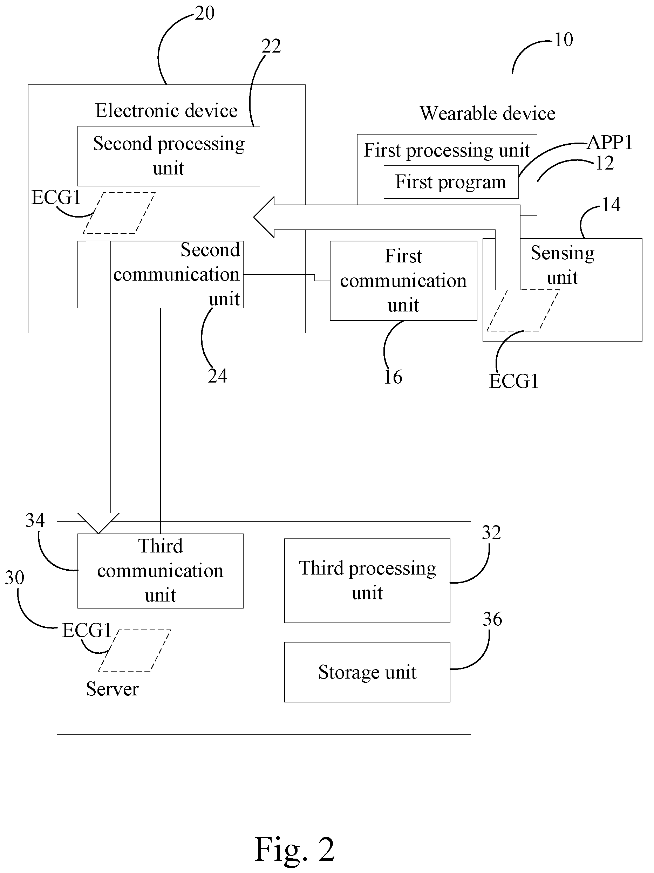

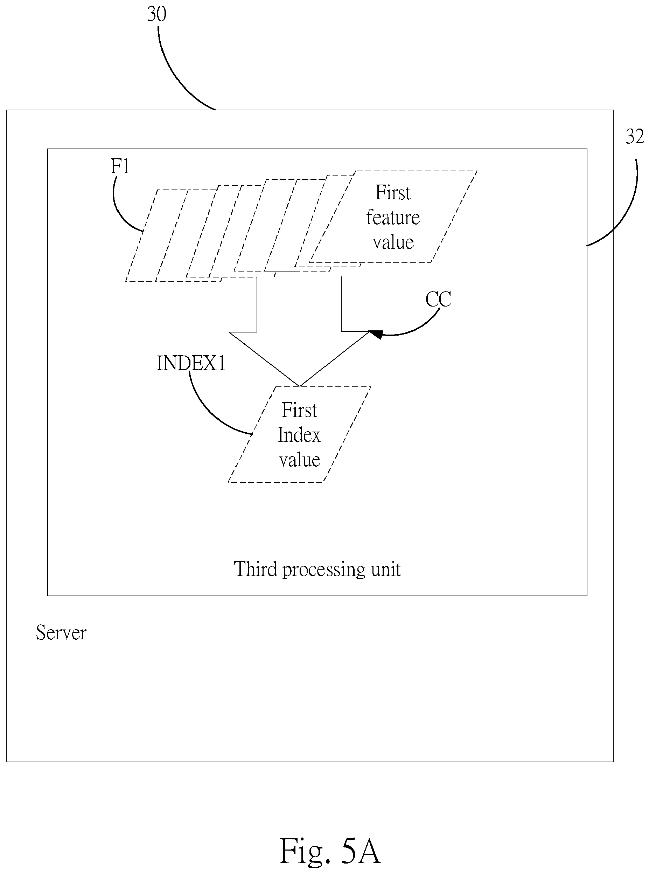

Refer to Fig. 2-5B, a system for transforming ECG signals into an index value according to the present application is revealed. A sensing device used in this embodiment is a wearable device 10. The system of this embodiment includes the wearable device 10, an electronic device 20, and a server 30. The wearable device 10 consists of a first processing unit 12, a sensing unit 14, and a first communication unit 16 while the electronic device 20 is composed of a second processing unit 22 and a second communication unit 24. The server 30 includes a third processing unit 32, a third communication unit 34, and a storage unit 36.

In this embodiment, the wearable device 10 can be a smartwatch, a smart band, or a vision-based wearable device such as Apple Watch, Xiaomi smart band, or virtual reality (VR) devices. Take the wearable VR device as an example. The wearable device 10 is not limited to the wearable VR device. It can also be a wearable augmented reality (AR) device, or a wearable mixed reality (MR) device. The electronic device 20 is a general consumer electronic such as a smart phone, a tablet, or a portable video and audio device. As to the server 30, it can be a large-scale computing device on the internet, a cloud server, or a remote device with cloud computing capability.

The method for transforming ECG signals into an index value according to the present application includes the steps S10-S30. The present method is applied to the wearable device 10 which is wirelessly connected with the electronic device 20 by the first communication unit 16 of the wearable device 10 and the second communication unit 24 of the electronic device 20 connected with each other wirelessly. The electronic device 20 is connected with the server 30 by the second communication unit 24 of the electronic device 20 connected with the third communication unit 34 of the server 30. In the step S05, as shown in Fig. 2, the first processing unit 12 of the wearable device 10 runs a first program APP1 and the sensing unit 14 senses a first ECG signal ECG 1. Then the first program APPA 1 uploads the first ECG signal ECG 1 to the server 30 through the electronic device 2.

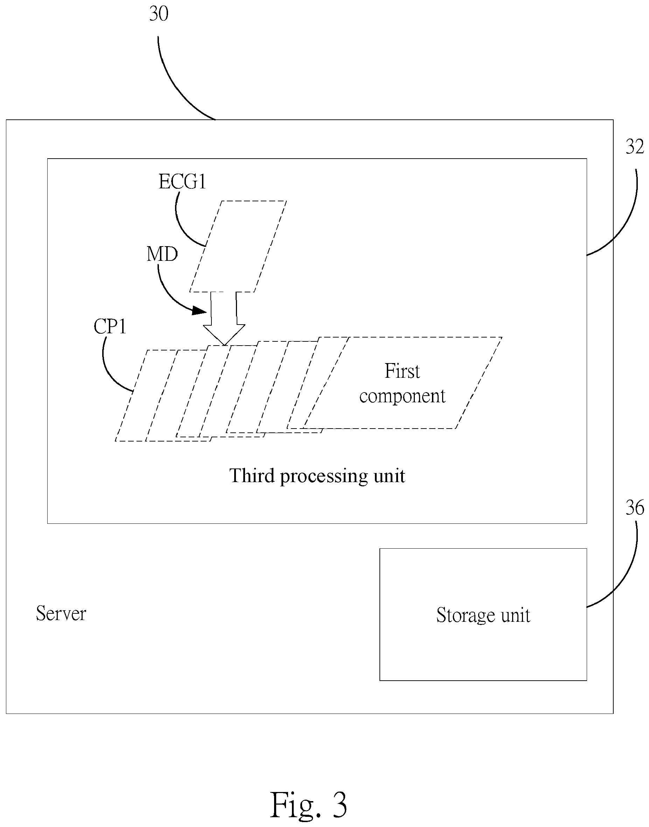

As shown in Fig. 3, in the step S10, the server 30 executes a decomposition MD using the third processing unit 32 and the first ECG signal ECG 1 is decomposed into a plurality of first components CP1. The third processing unit 32 performs decomposition MD based on an Ensemble Empirical Mode Decomposition (EEMD) to decompose the first ECG signal ECG 1 in analog form into the first components CP1. That means the first ECG signal ECG 1 is considered as $x(t) = \sum_{i=1}^{n} c_i(t)$ and decomposed into several components $c_1(t)$, $c_2(t)$.... $c_n(t)$ based on their energy distribution.

After completing the decomposition MD, the third processing unit 32 further performs clustering and classification of the first components CP1. For example, the first components CP1 are divided into major waves, QRS complex, and minor waves PQST. The normal electrocardiogram (ECG) is formed by P wave, PR segment, QRS complex, ST segment, T wave, and U wave. The QRS complex represents the depolarization of the left and right ventricles of the heart. The ventricles contain more muscle mass than other parts of the heart so that the QRS complex is considerably larger than other waves. Thus, the QRS complex gets a better result of the component after the mode decomposition.

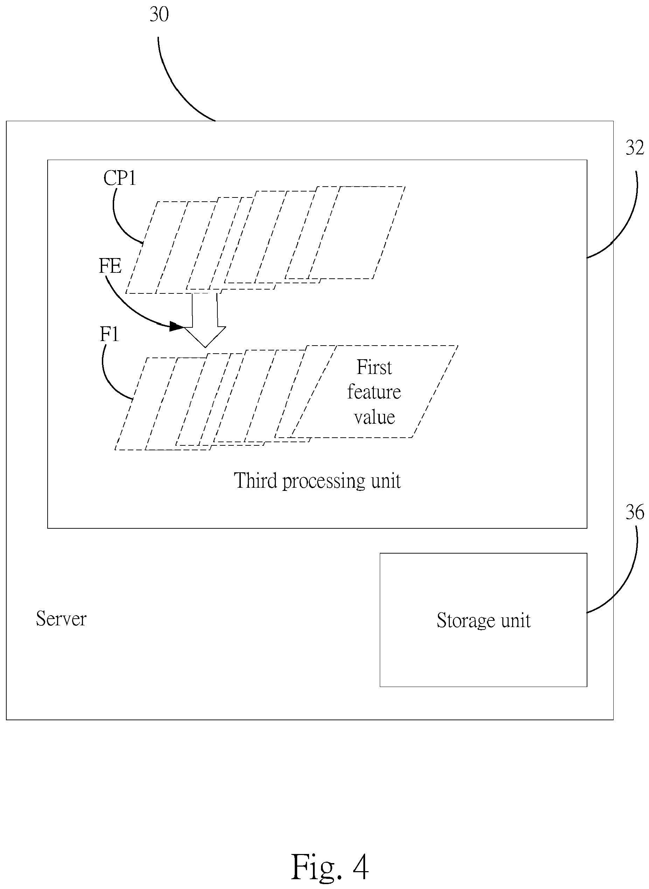

Refer to Fig. 4, in the decomposition of step S20, the server 30 runs a feature extraction (FE) using the third processing unit 32. The feature extraction from the first components CP1 is carried out to generate a plurality of first feature values F1. For example, the third processing unit 32 runs the feature extraction FE according to principal component analysis (PCA) to get the first feature values F1 from the first components CP1 by removing excessive repetitive components and keeping the components with greater variance. Since computing results of this embodiment are represented by energy levels, the repetitive components should be removed. Besides, the feature extraction FE can also be performed by the third processing unit 32 according to respective time-frequency energy distribution of the components of the major waves (QRS complex) and minor waves PQST and subtle correlation and variance between the two groups of the waves. Thereby the first feature values F1 are obtained.

As shown in Fig. 5A, in the step S30, the server 30 performs geometric computation CC to generate a first index value INDEX1 according to the first feature values F1 by using the third processing unit 32. For example, the first feature values F1 are combined and the first index value INDEX1 is obtained by vector relationship among the first feature values F1. That means a unique solution which is obtained by the geometric computation CC of the first feature values F1 is the first index value INDEX1. In the step S30, the first index value INDEX1 can be normalized by the third processing unit 32 to be within 0.0 and 10.0.

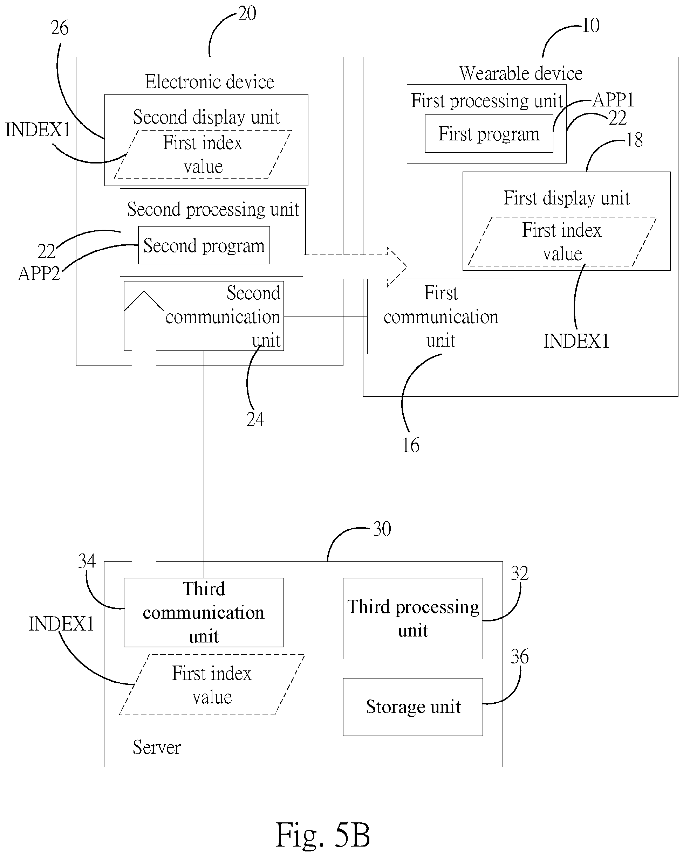

In this embodiment, the method further includes the step S35. As shown in Fig. 5B, the first index value INDEX1 is sent back to the electronic device 20 by the third communication unit 34 of the server 30. Then the first index value INDEX1 is shown on a screen of a second display unit 26 of the electronic device 20 or further transmitted from the electronic device 20 to the wearable device 10 by the electronic device 20 and displayed on a screen of a first display unit 18 of the wearable device 10. Thereby a simple numerical value is provided so that users can catch their body (health) conditions according to a magnitude of the numerical value shown on the wearable device 10 or the electronic device 20.

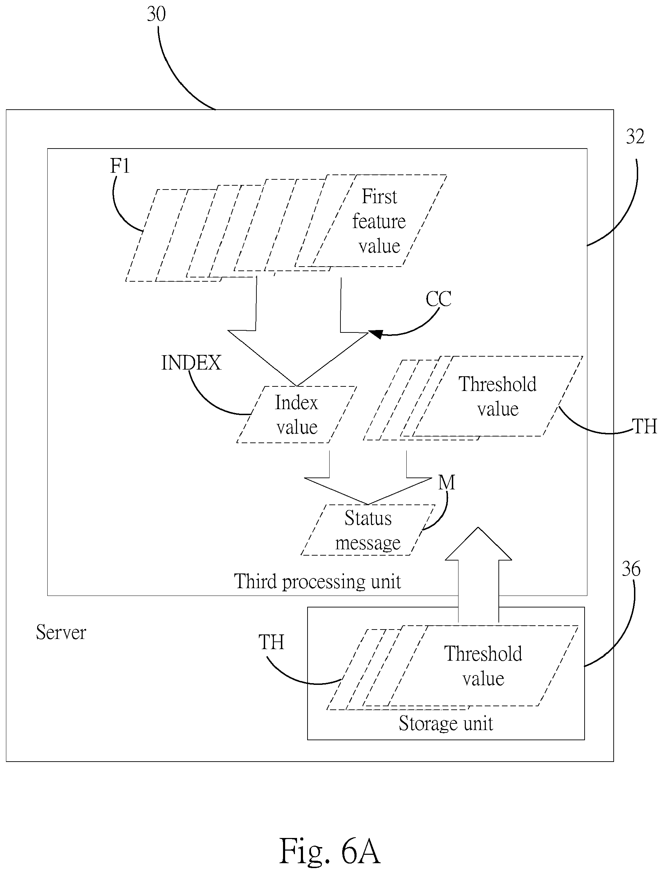

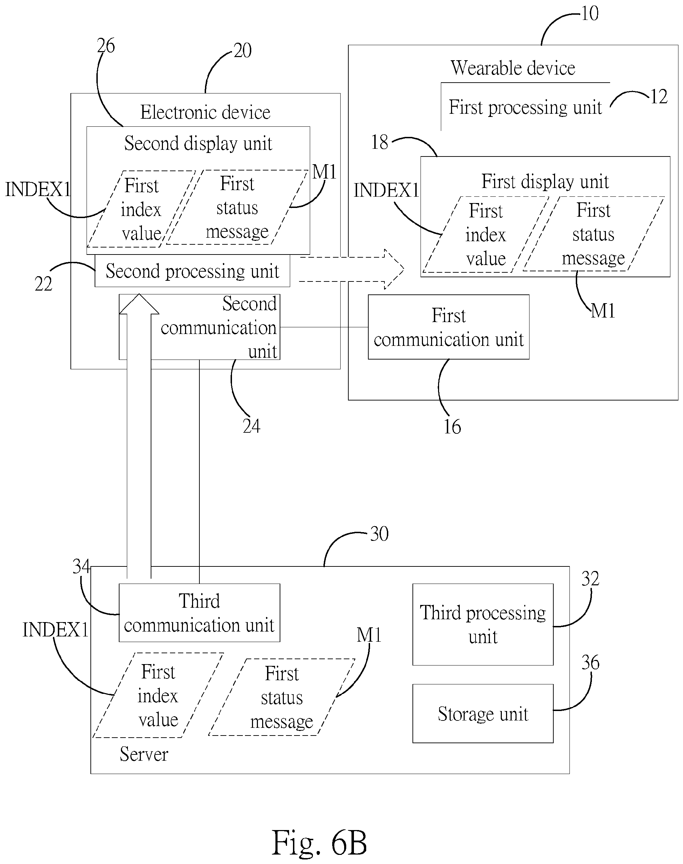

Moreover, as the step S30 shown in Fig. 6A, the third processing unit 32 of the server 30 reads a plurality of threshold values TH from a database DB and generates a first status message M1 according to the threshold values TH and the first index value INDEX1. For instance, the threshold values TH are 6, 5, 4, and 0. The first status message M1 is "Well" when the first index value INDEX1 is no less than 6. The first status message M1 is "Ordinary" when the first index value INDEX1 is 5 or between 5 and 6 ($5 \leq$ INDEX1 <6). The first status message M1 is "Beware" when the first index value INDEX1 is 4 or between 4 and 5 ($4 \leq$ INDEX1 <5). The first status message M1 is "Caution" when the first index value INDEX1 is between 0 and 4. The first status message M1 is "Alert" when the first index value INDEX1 is smaller than 0. In the step S35, as shown in Fig. 6B, the first status message M1 corresponding to the first index value INDEX1 is further displayed on the first display unit 18 or the second display unit 26. The message of Caution or Alert further includes content that suggests users to seek medical attention as soon as possible. In the message of Caution, the user is suggested to see a doctor for follow up while the Alert message suggests the user to seek medical help immediately.

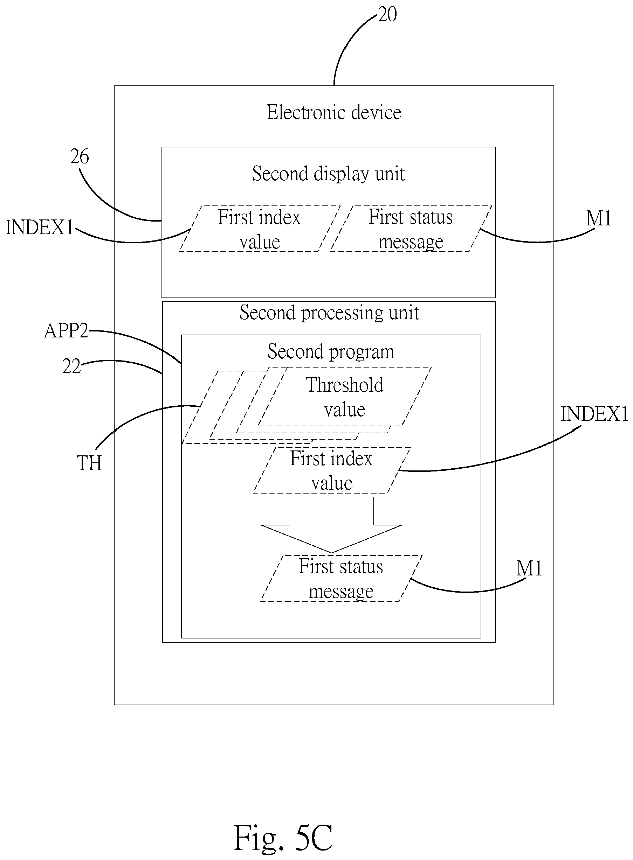

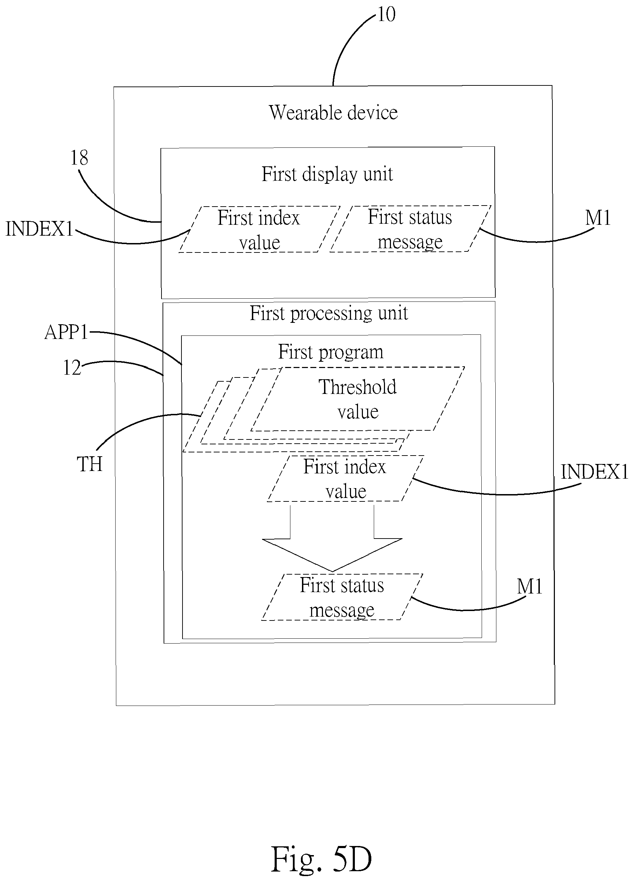

Furthermore, as shown in Fig. 5C, another embodiment is revealed. The electronic device 20 executes a second program APP2 to generate the first status message M1 according to threshold values TH preset in the electronic device 20 and the first index value INDEX1 and then the first status message M1 is shown on the second display unit 26. In other words, the threshold values TH which are stored in the electronic device 20 are read by the second processing unit 22 of the electronic device 20 to generate the first status message M1 according to the threshold values TH and the first index value INDEX1. Or as shown in Fig. 5D, the first program APP1 run by the first processing unit 12 of the wearable device 10 generates the first status message M1 according to threshold values TH preset in the wearable device 10 and the first index value INDEX1 in a further embodiment. Then the first status message M1 is shown on the first display unit 18 under control of the first program APP1. That is to say the first program APP1 generates the corresponding first status message M1 according to the threshold values TH which are stored in the wearable device 10.

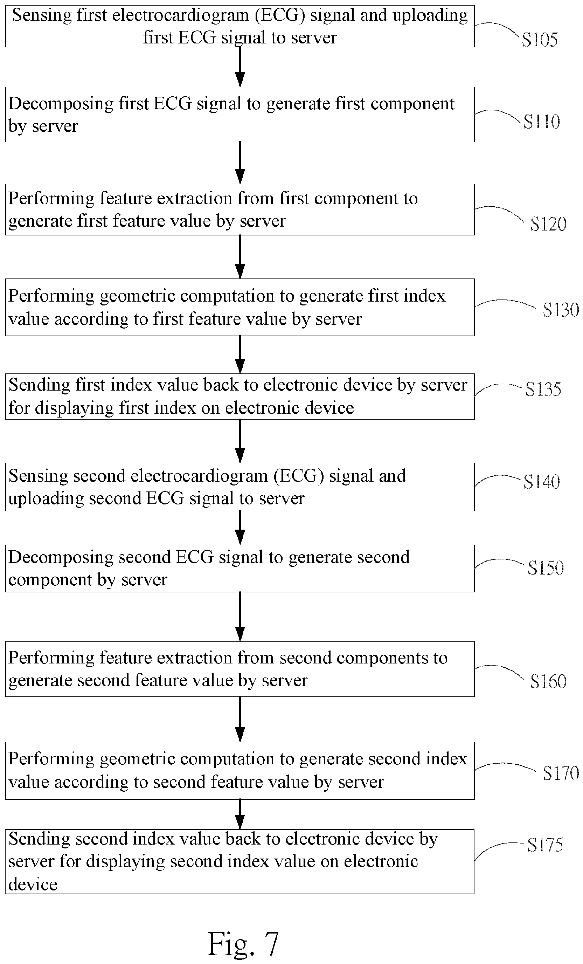

Refer to Fig. 7, a flow chart of another embodiment is revealed. The difference between the embodiment in Fig. 1 and the embodiment in Fig. 7 is in that this embodiment in Fig. 7 further includes steps of sensing a second electrocardiogram (ECG) signal to get a second index value INDEX2. In the embodiment shown in Fig. 7, the sensing device used is still a wearable device 10.

A method for transforming ECG signals into an index value of this embodiment according to the present application includes the following steps.

Step S105: Sensing first electrocardiogram (ECG) signal and uploading first ECG signal to server;

Step S110: Decomposing first ECG signal to generate first component by server;

Step S120: Performing feature extraction from first component to generate first feature value by server;

Step S130: performing geometric computation to generate first index value according to first feature value by server;

Step S135: Sending first index value back to electronic device by server for displaying first index on electronic device;

Step S140: Sensing second electrocardiogram (ECG) signal and uploading second ECG signal to server;

Step S150: Decomposing second ECG signal to generate second component by server;

Step S160: Performing feature extraction from second components to generate second feature value by server;

Step S170: Performing geometric computation to generate second index value according to second feature value by server;

Step S175: Sending second index value back to electronic device by server for displaying second index value on electronic device.

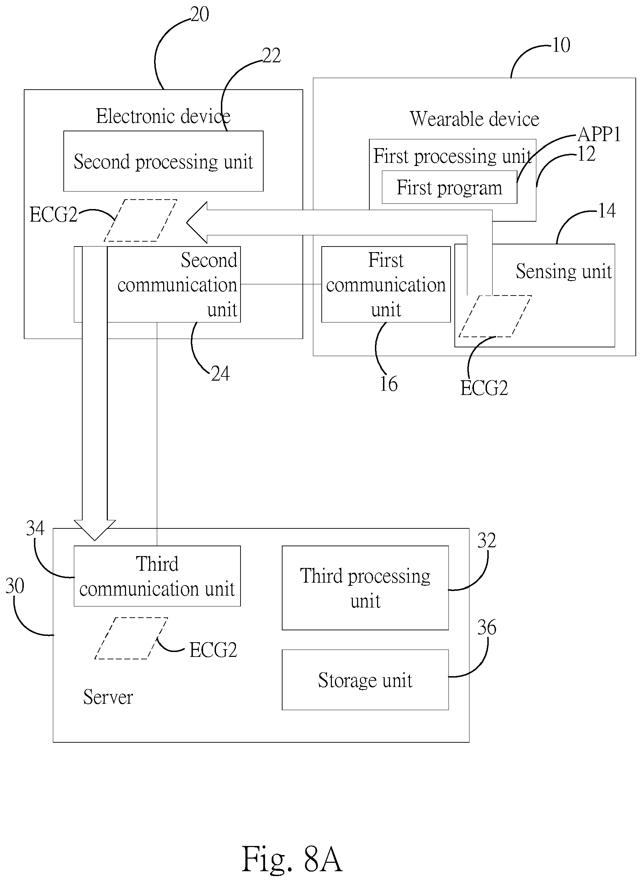

The steps S105-S135 are the same as the above steps S05-S35. As shown in Fig. 8A, the sensing unit 14 of the wearable device 10 further senses a second electrocardiogram (ECG2) signal of a user with the wearable device 10. Then the second ECG2 signal is sent to the second communication unit 24 by the first program (APP1) using the first communication unit 16 and further sent from the second communication unit 24 to the third communication unit 34. Thereby the second ECG2 signal is sent to the server 30,

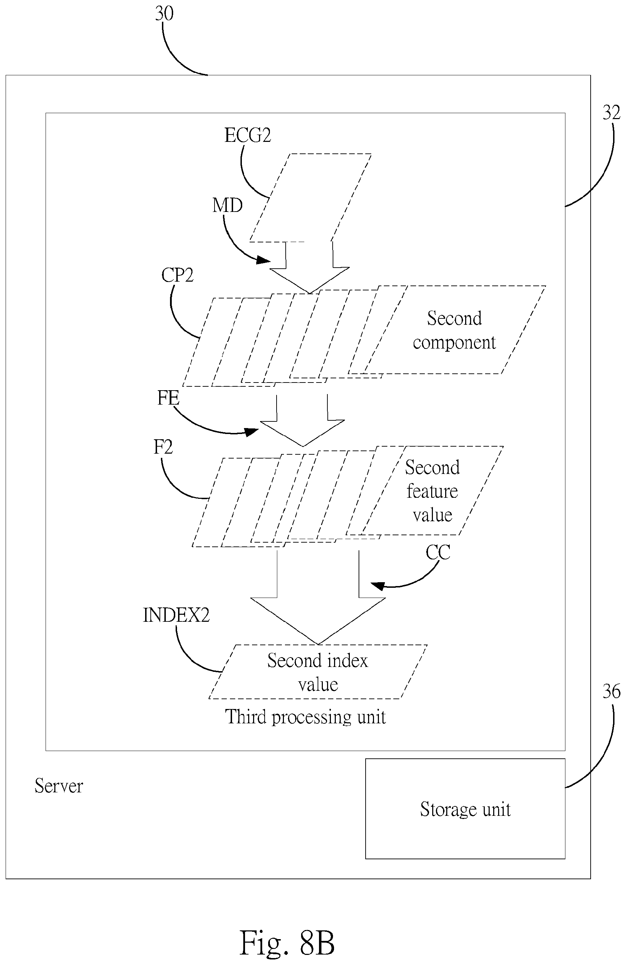

Refer to Fig. 8B, in the step S 150, the server 30 executes the decomposition MD using the third processing unit 32 and the second ECG signal ECG 2 is decomposed into a plurality of second components CP2. Then, the feature extraction from the second components CP2 is carried out by the third processing unit 32 to get a plurality of second feature values F2 in the step S160. Next in the step S170, the third processing unit 32 combines the second feature values F2 by the geometric computation CC to get a second index value INDEX2.

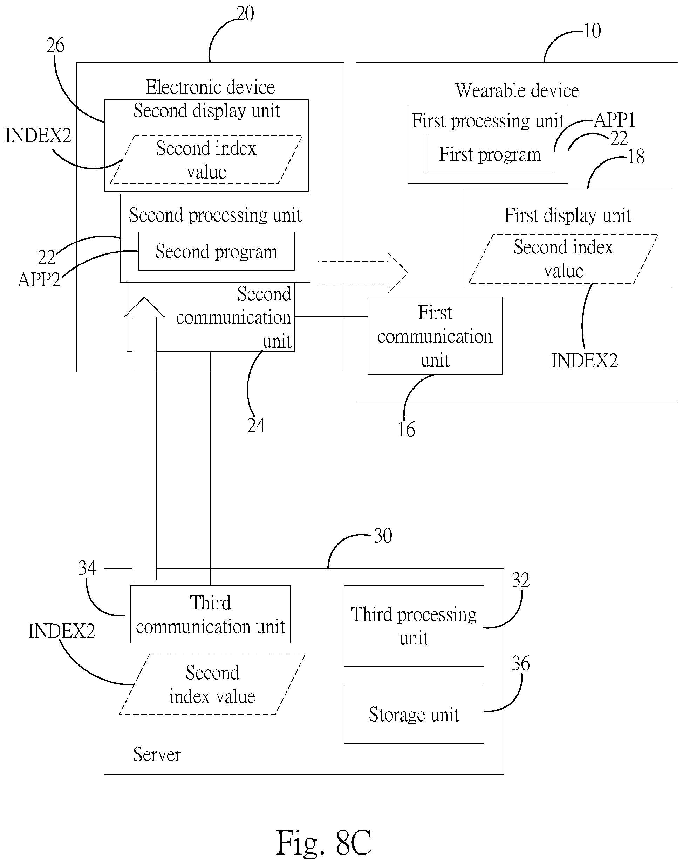

As shown in Fig. 8C, in the step S175, the server 30 uses the third communication unit 34 to send the second index value INDEX2 back to the electronic device 20 for direct display of the second index value INDEX2 on the electronic device 20. The second index value INDEX2 can also be shown on the wearable device 10.

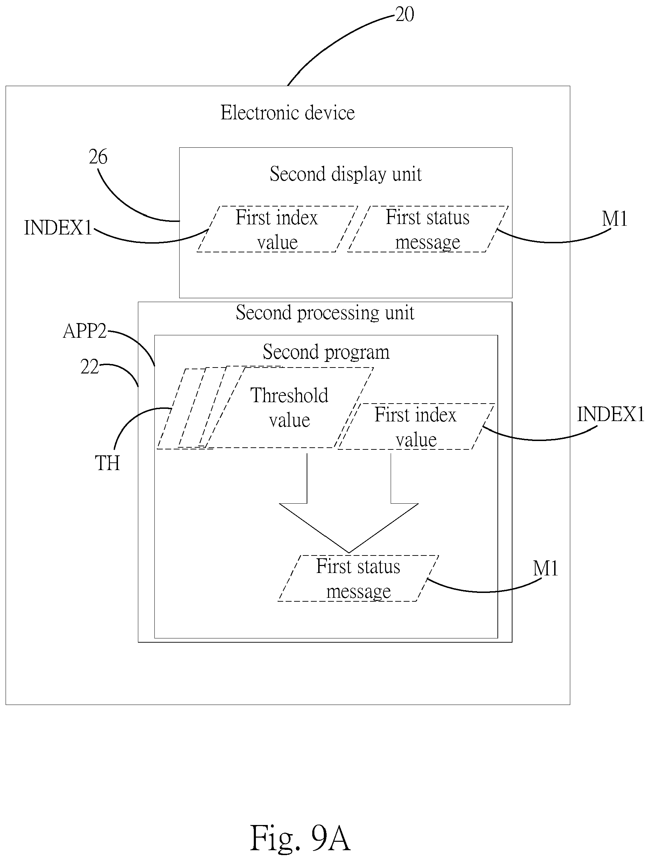

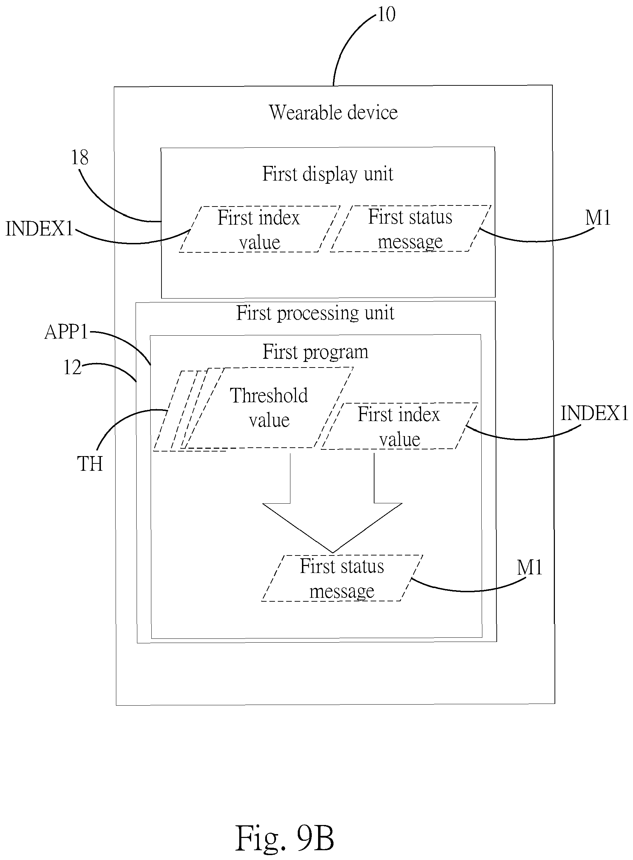

Refer to Fig. 9A, in a further embodiment, the electronic device 20 runs the second program APP2 to generate a second status message M2 according to the threshold values TH and the second index value INDEX2 and then the second status message M2 is shown on the second display unit 26. Or in a further embodiment shown in Fig. 9B, the first processing unit 12 of the wearable device 10 executes the first program APP1 to generate the second status message M2 according to the threshold values TH and the second index value INDEX2 and then the second status message M2 is shown on the first display unit 18.

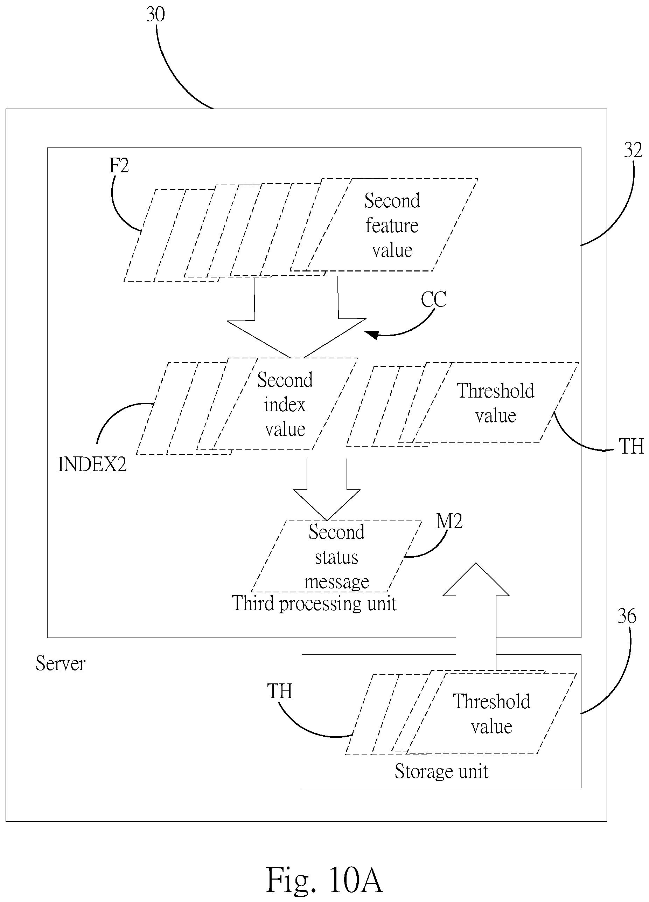

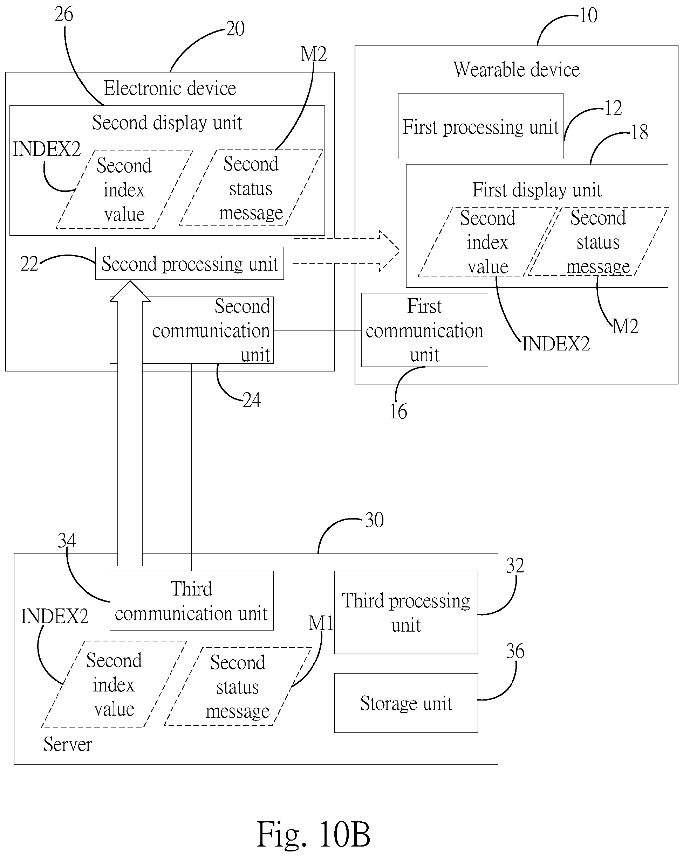

Refer to Fig. 10A, in a further embodiment, besides generating the second index value INDEX2, the third processing unit 32 of the server 30 also reads a plurality of threshold values TH from a database DB and generates a second status message M2 according to the threshold values TH and the second index value INDEX2 in the step S170. In the step S175, as shown in Fig. 10B, the server 30 sends the second index value INDEX2 and the second status message M2 back to the electronic device 20. Then the second index value INDEX2 and the corresponding second status message M2 can be further displayed on either the first display unit 18 or the second display unit 26.

In the above embodiments of a method and a system for transforming electrocardiogram (ECG) signals into an index value, an iPhone (electronic device 20) and an APPLE Watch (wearable device 10) are provided to a user. The APPLE Watch runs a first program APP1 to sense the first ECG signal ECG1 and the second ECG signal ECG2 of the user separately. Then the first ECG signal ECG1 before exercise and the second ECG signal ECG2 after exercise are uploaded to the server 30 associated with the first program APP1 through the iPhone. Then the server 30 performs decomposition of the first ECG signal ECG1 and the second ECG signal ECG2 separately to get a plurality of first components CP1 and a plurality of second components CP2 respectively and correspondingly. The server 30 further performs clustering and classification of the first components CP1 and the second components CP2 to form major waves QRS complex and minor waves PQST. Next a plurality of first feature values F1 and a plurality of second feature values F2 are obtained according to the first components CP1 and the second components CP2 as well as the corresponding major waves QRS complex and the corresponding minor waves PQST. Then the third processing unit 32 performs geometric computation CC of the first feature values F1 and the second feature values F2 in batches to generate the first index value INDEX1 and the second index value INDEX2. Therefore, the heart's response to different exercise doesn't to be measured at different stages and the patient's physical burden is relieved.

Moreover, the third processing unit 32 of the server 30 generates the first status message M1 and the second status message M2 respectively corresponding to the first index value INDEX1 and the second index value INDEX2 according to the threshold values TH. Once the first index value INDEX1 and the second index value INDEX2 are respectively 5.2 and 5.4, the first status message M1 and the second status message M2 are both corresponding to "Ordinary". Then the first and the second index values INDEX1, INDEX2 and the corresponding first and second status messages M1, M2 are provided and transmitted to the electronic 20 correspondingly or further to the wearable device 10 to be displayed thereon.

After receiving the first index value INDEX1 and the second index value INDEX2 separately, the second program APP2 of the electronic device 20 generates the first status message M1 and the second status message M2 respectively corresponding to the first index value INDEX1 and the second index value INDEX2 according to the threshold values TH. Then the first and the second index values INDEX1, INDEX2 and the corresponding first and second status messages M1, M2 are separately shown on the electronic device 20. Moreover, the wearable device 10 uses the first processing unit 12 to generate the first status message M1 and the second status message M2 respectively corresponding to the first index value INDEX1 and the second index value INDEX2 according to the threshold values TH and the first and second index values INDEX1, INDEX2 after receiving the first index value INDEX1 and the second index value INDEX2 separately. Then the first and the second index values INDEX1, INDEX2 and the corresponding first and second status messages M1, M2 are separately shown on the wearable device 10. Thereby people can obtain changes in their health conditions directly by the first index value INDEX1 and the second index value INDEX2, without reading (interpreting) complex ECG. Or the present method and system can be applied to simplified exercise ECG for measurement of ECG signals before and after exercise.

The following embodiments are provided with different sensing devices.

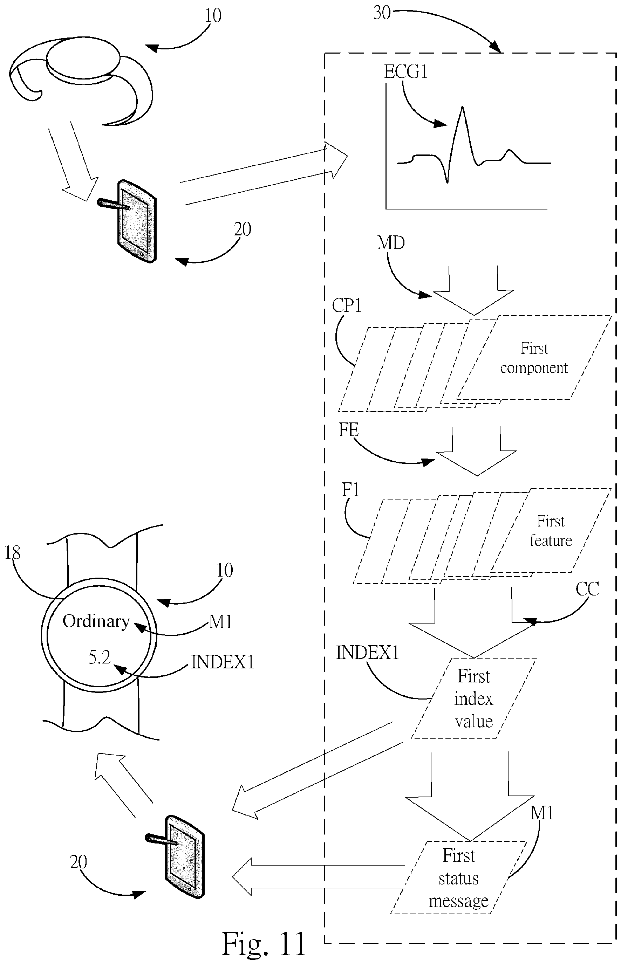

Refer to Fig. 1-11, take a smartwatch used as the wearable device 10 as an example and the following is detailed description.

A user wears an Apple Watch (the wearable device 10 mentioned above), a processor of the Apple Watch (which is equal to the first processing unit 12 of the wearable device 10) senses a user's arm using a sensing unit 14 of the APPLE watch. That means a single lead is used to measure a first ECG signal ECG1 of the user. The first program APP1 run on the Apple Watch transmits the first ECG signal ECG1 to the server 30 by the electronic device 20. Then the server 30 performs decomposition MD, feature extraction FE, and geometric computation CC on the first ECG signal ECG1. Thus, the first ECG signal ECG1 is decomposed into a plurality of first components CP1 from which a plurality of first feature values F1 is further obtained. Thereby a corresponding first index value INDEX1 is obtained and used for allowing the user to catch his/her heart status.

Furthermore, the server 30 can send the first index value INDEX1 back to the electronic device 20 so that the Apple Watch or the electronic device 20 shows the first index value INDEX1. The server 30 further compares the first index value INDEX1 with at least one threshold value TH to get the first status message M1. That means the server 30 provides the first index value INDEX1 and the corresponding first status message M1 to the electronic device 20 and the Apple Watch (the wearable device 10) connected to the electronic device 20 can also read the first index value INDEX1 and the corresponding first status message M1 and show them on the first display unit 18.

Besides the corresponding first status message M1 provided by the server 30, the second program APP2 run in the electronic device 20 can also compare the first index value INDEX1 with at least one threshold value TH to get the first status message M1 and show the first status message M1 on the second display unit 26 of the electronic device 20. Or the first program APP1 executed by the wearable device 10 compares the first index value INDEX1 with at least one threshold value TH to get the first status message M1 and show the first status message M1 on the first display unit 18.

When the user sees the first index value INDEX1 which is 6.8 and the corresponding first status message M1 is "Well", he has no worries about the heart status. But once the first index value INDEX1 displayed is 3.8 and the corresponding first status message M1 is "Caution", the user needs to worry about whether he should see a doctor and further get checked by cardiologists in order to catch heart conditions. Therefore, the user can catch his/her heart status directly by means of the first index value INDEX1 and the corresponding first status message M1, without the need to interpret waveforms of the ECG1 and find out any abnormal signs related to the heart status.

Additionally, users can measure ECG signals at different times by using the wearable device 10. For example, the wearable device 10 is used to measure different ECG signals such as the first ECG signal ECG1 and the second ECG signal ECG2 before and after the mood swings. Then different index values INDEX1, INDEX2 are obtained after transformation by the present method and users can catch changes in the heart status according to the index values, such as the first and second index values INDEX1, INDEX2. When the index values INDEX1, INDEX2 are increased such as from 5.2 to 5.8, the corresponding heart status is fine. In contrast, when the first and second index values INDEX1, INDEX2 are reduced such as from 5.2 to 3.8, the corresponding heart status is cautious. That means the user needs to be careful about the health of the heart and thinking about whether to seek medical help or not. Thereby the user can catch the heart status directly by the first and second index values INDEX1, INDEX2 represented visually. The method can also be applied to measurement of ECG signals before and after exercise, treatment, sleep or any other things which may affect cardiovascular health and cause changes in the ECG signals to get corresponding index values and messages showing the heart status.

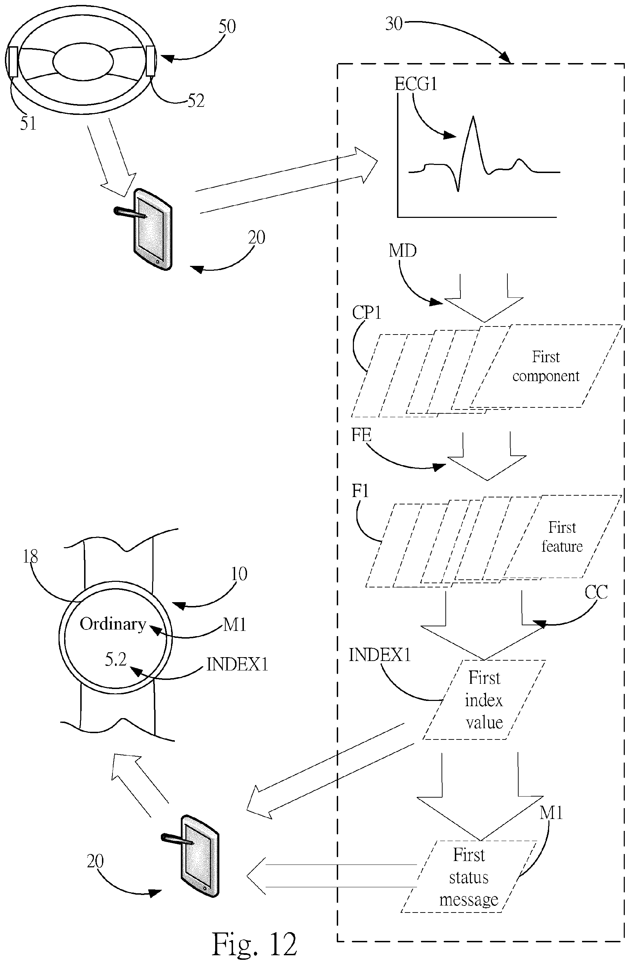

The sensing device used is not limited to the wearable device 10. As shown in Fig. 12, the sensing device can be two grip sensors 51, 52 on a steering wheel 50. Most of in-vehicle devices available now support Bluetooth so that the electronic device 20 of this embodiment is connected with the grip sensors 51, 52 by Bluetooth wireless transmission. Then the two grip sensors 51, 52 detect the first ECG signal ECG1 and transmits the first ECG signal ECG1 to the electronic device 20 by Bluetooth. The first ECG signal ECG1 is further sent to the server 30 and processed by decomposition MD, feature extraction FE, and geometric computation CC to get the corresponding first index value INDEX1. Next the electronic device 20 generates the corresponding first status message M1 according to the first index value INDEX1 provided by the server 30 and the threshold values TH stored in itself. Besides the electronic device 20, the first index value INDEX1 and the corresponding first status message M1 can also be shown on the wearable device 10.

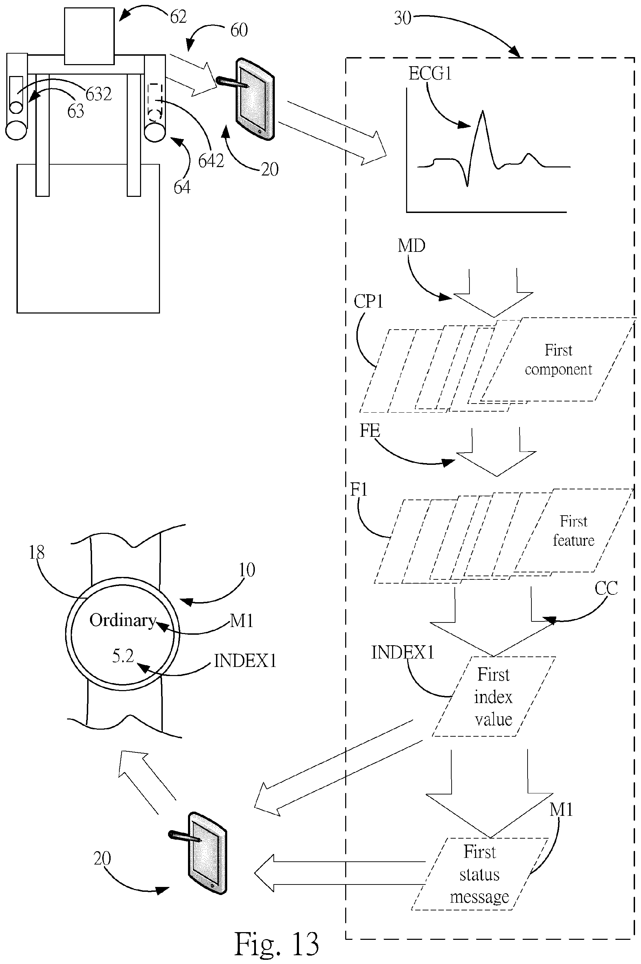

In addition, the present method can be applied to treadmills. A control panel 62 of a treadmill 60 can be wirelessly connected with the electronic device 20. Refer to Fig. 13, the sensing device used in this embodiment are two grip sensors 632, 642 on two hand grips 63, 64 of the treadmill 60. How the rest components are connected and how the first index value INDEX1 and the corresponding first status message M1 are generated are the same as those of the above embodiment so that they are not described in detail herein.

According to above detailed description, the present application provides following characteristics,

1. No artificial intelligence operation;

2. Unique index value by each time operation for ECG signal;

3. Status Message corresponds to the actual status of the users.

In summary, a method for transforming electrocardiogram (ECG) signals into index values and a system thereof according to the present application are used to obtain a corresponding index value by decomposition MD, feature extraction FE, and geometric computation CC of ECG signals. Then a corresponding status message is obtained by comparing the index value with the threshold values. Thus, the users can catch their own health status directly by the index value in combination with the status message, without reading complex waveforms of the ECG signals.

Additional advantages and modifications will readily occur to those skilled in the art. Therefore, the invention in its broader aspects is not limited to the specific details, and representative devices shown and described herein. Accordingly, various modifications may be made without departing from the spirit or scope of the general inventive concept as defined by the appended claims and their equivalent.

**Claims**

1. A method for transforming electrocardiogram (ECG) signals into an index value, which is applied to a sensing device (10 , 51 , 52) used for detecting a first ECG signal (ECG1) and wirelessly connected with an electronic device (20) while the first ECG signal (ECG1) is uploaded to a server (30) through the electronic device (20) by a first program, comprising the steps of:

   decomposing the first ECG signal (ECG1) to generate a plurality of first components (CP1) by the server (30);
   performing a feature extraction (FE) from the first components (CP1) to generate a plurality of first feature values (F1) by the server (30); and
   performing geometric computation (CC) to generate a first index value (INDEX1) according to the first feature values (F1) by the server (30).

2. The method as claimed in claim 1, wherein in the step of performing geometric computation (CC) to generate a first index value (INDEX1) according to the first feature values (F1) by the server (30), the server (30) is set with a plurality of threshold values (TH) corresponding to the first index value (INDEX1) and the server (30) generates a first status message (M1) according to the threshold values (TH) and the first index value (INDEX1).

3. The method as claimed in claim 1, wherein after the step of performing geometric computation (CC) to generate a first index value (INDEX1) according to the first feature values (F1), the method further includes a step of sending the first index value (INDEX1) back to the electronic device (20) by the server (30) to display the first index value (INDEX1) on the electronic device (20).

4. The method as claimed in claim 3, wherein the electronic device (20) is set with a plurality of threshold values (TH) corresponding to the first index value (INDEX1); the electronic device (20) generates a first status message (M1) according to the threshold values (TH) and the first index value (INDEX1); the first index value (INDEX1) and the first status message (M1) are shown on the sensing device (10 , 51 , 52) or the electronic device (20).

5. The method as claimed in claim 1, wherein the sensing device (10 , 51 , 52) further detects a second ECG signal (ECG2) and uploads the second ECG signal (ECG2) to the server (30) through the electronic device (20) and the method further includes the steps of:

   decomposing the second ECG signal (ECG2) to generate a plurality of second components (CP2) by the server (30);
   performing the feature extraction (FE) from the second components (CP2) to generate a plurality of second feature values (F2) by the server (30); and
   performing geometric computation (CC) to generate a second index value (INDEX2) according to the second feature values (F2) by the server (30).

6. The method as claimed in claim 5, wherein in the step of performing geometric computation (CC) to generate a second index value (INDEX2) according to the second feature values (F2) by the server (30), the server (30) is set with a plurality of threshold values (TH) corresponding to the second index value (INDEX2); the server (30) further generates a second status message (M2) according to the threshold values (TH) and the second index value (INDEX2).

7. The method as claimed in claim 5, wherein the method further includes a step of: sending the second index value (INDEX2) back to the electronic device (20) for displaying the second index value (INDEX2) on the electronic device (20).

8. The method as claimed in claim 7, wherein the electronic device (20) is set with a plurality of threshold values (TH) corresponding to the second index value (INDEX2) and the electronic device (20) generates a second status message (M2) according to the threshold values (TH) and the second index value (INDEX2); the second status message (M2) is shown on the sensing device (10 , 51 , 52) or the electronic device (20).

9. A system for transforming electrocardiogram (ECG) signals into an index value, comprising:

   a sensing device (10 , 51 , 52) provided with a sensing unit (14) for sensing a first electrocardiogram (ECG) signal;

an electronic device (20) which is wirelessly connected with the sensing device (10 , 51 , 52) and used for receiving the first ECG signal (ECG1); and

a server (30) connected with the electronic device (20) which uploads the first ECG signal (ECG1) to the server (30); the first ECG signal (ECG1) is decomposed to generate a plurality of first components (CP1) by the server (30); then the server (30) performs a feature extraction (FE) from the first components (CP1) to get a plurality of first feature values (F1); next the server (30) performs geometric computation (CC) to generate a first index value (INDEX1) according to the first feature values (F1).

10. The system as claimed in claim 9, wherein the server (30) is set with a plurality of threshold values (TH) corresponding to the first index value (INDEX1) and the server (30) generates a first status message (M1) according to the threshold values (TH) and the first index value (INDEX1); the server (30) sends the first index value (INDEX1) and the first status message (M1) back to the electronic device (20) and the first index value (INDEX1) and the first status message (M1) are shown on the electronic device (20) or the sensing device (10 , 51 , 52).

11. The system as claimed in claim 9, wherein the server (30) sends the first index value (INDEX1) back to the electronic device (20).

12. The system as claimed in claim 11, wherein the electronic device (20) is set with a plurality of threshold values (TH) corresponding to the first index value (INDEX1); the electronic device (20) generates a first status message (M1) according to the threshold values (TH) and the first index value (INDEX1); the first index value (INDEX1) and the first status message (M1) are shown on the electronic device (20) or the sensing device (10 , 51 , 52).

13. The system as claimed in claim 7, wherein the sensing unit (14) further senses a second ECG signal (ECG2) and the electronic device (20) uploads the second ECG signal (ECG2) to the server (30); the second ECG signal (ECG2) is decomposed to generate a plurality of second components (CP2) by the server (30); then the server (30) performs the feature extraction (FE) from the second components (CP2) to get a plurality of second feature values (F2); next the server (30) performs geometric computation (CC) to generate a second index value (INDEX2) according to the second feature values (F2).

Sensing first electrocardiogram (ECG) signal and uploading first ECG signal to server — S05

Decomposing first ECG signal to generate first component by server — S10

Performing feature extraction from first component to generate first feature value by server — S20

Performing geometric computation to generate first index value according to first feature value by server — S30

Sending data back to electronic device by server for displaying data on electronic device — S35

Fig. 1

Fig. 2

30

32

ECG1

MD

CP1

First
component

Third processing unit

36

Server

Storage unit

Fig. 3

30

32

CP1

FE

F1

First
feature
value

Third processing unit

36

Server

Storage unit

Fig. 4

30

32

F1

First feature value

CC

INDEX1

First Index value

Third processing unit

Server

Fig. 5A

26

20

Electronic device

INDEX1

Second display unit

First index
value

Second processing unit

22
APP2

Second program

Second
communication
unit

24

10

Wearable device

First processing unit

APP1

First program

22

18

First display unit

First index
value

INDEX1

First
communication
unit

16

30

34

INDEX1

Third
communication
unit

First index
value

Server

Third processing
unit

32

36

Storage unit

Fig. 5B

Fig. 5C

10

Wearable device

18

First display unit

INDEX1

First index value  First status message  M1

First processing unit

APP1

12

First program

Threshold value

TH

First index value  INDEX1

First status message  M1

Fig. 5D

Fig. 6A

Fig. 6B

Sensing first electrocardiogram (ECG) signal and uploading first ECG signal to server — S105

Decomposing first ECG signal to generate first component by server — S110

Performing feature extraction from first component to generate first feature value by server — S120

Performing geometric computation to generate first index value according to first feature value by server — S130

Sending first index value back to electronic device by server for displaying first index on electronic device — S135

Sensing second electrocardiogram (ECG) signal and uploading second ECG signal to server — S140

Decomposing second ECG signal to generate second component by server — S150

Performing feature extraction from second components to generate second feature value by server — S160

Performing geometric computation to generate second index value according to second feature value by server — S170

Sending second index value back to electronic device by server for displaying second index value on electronic device — S175

Fig. 7

Fig. 8A

Fig. 8B

Fig. 8C

Fig. 9A

Fig. 9B

30

F2

Second
feature
value

32

CC

Second
index
value

Threshold
value

TH

INDEX2

Second
status
message

M2

Third processing unit

Server

TH

Threshold
value

36

Storage unit

Fig. 10A

Fig. 10B

Fig. 11

50

52

51

20

30

ECG1

MD

CP1

First
component

FE

F1

First
feature

CC

INDEX1

First
index
value

INDEX1

First
status
message

M1

18

10

Ordinary

M1

5.2

INDEX1

20

Fig. 12

Fig. 13

Europäisches
Patentamt

European
Patent Office

Office européen
des brevets

# EUROPEAN SEARCH REPORT

**Application Number**

EP 23 18 0353

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/397313 A1 (ATTIA ITZHAK ZACHI [US] ET AL) 24 December 2020 (2020-12-24) * paragraphs [0002], [0006], [0056]; claims 1, 12, 16; figures 1, 2 * | 1-13 | INV. A61B5/00 A61B5/024 A61B5/332 A61B5/346 |
| X | US 2019/269344 A1 (SHAH RAKESH [US]) 5 September 2019 (2019-09-05) * paragraphs [0002], [0089], [0090]; claims 1, 10; figures 1, 22 * | 1,9 | |
| X | US 9 042 970 B2 (LU YING-CHIANG [SG]; TAN KAE YUAN [SG] ET AL.) 26 May 2015 (2015-05-26) * claims 1, 10; figure 1 * | 1,9 | |
| A | WANG CHENYU ET AL: "A Low Power Cardiovascular Healthcare System With Cross-Layer Optimization From Sensing Patch to Cloud Platform", IEEE TRANSACTIONS ON BIOMEDICAL CIRCUITS AND SYSTEMS, IEEE, US, vol. 13, no. 2, 11 January 2019 (2019-01-11), pages 314-329, XP011715969, ISSN: 1932-4545, DOI: 10.1109/TBCAS.2019.2892334 [retrieved on 2019-03-22] * abstract; figure 1 * | 1,9 | |
| A | CA 2 966 180 A1 (IRHYTHM TECH INC [US]) 6 May 2016 (2016-05-06) * paragraphs [0003], [0128], [0133], [0134], [0141], [0149]; figure 10 * | 1,9 | |

**TECHNICAL FIELDS SEARCHED (IPC)**

A61B
G16H

The present search report has been drawn up for all claims

| Place of search | Date of completion of the search | Examiner |
|---|---|---|
| The Hague | 9 October 2023 | Fauché, Yann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 18 0353

This annex lists the patent family members relating to the patent documents cited in  the above-mentioned European search report.
The members are as contained in the European Patent Office EDP file on
The European Patent Office is in no way liable for these particulars which are merely given for the purpose of information.

09-10-2023

| Patent document cited in search report | | Publication date | Patent family member(s) | | Publication date |
|---|---|---|---|---|---|
| US 2020397313 | A1 | 24-12-2020 | AU | 2018346412 A1 | 30-04-2020 |
| | | | CA | 3078519 A1 | 11-04-2019 |
| | | | CN | 111432720 A | 17-07-2020 |
| | | | EP | 3691524 A1 | 12-08-2020 |
| | | | JP | 7262452 B2 | 21-04-2023 |
| | | | JP | 2020536629 A | 17-12-2020 |
| | | | JP | 2023089112 A | 27-06-2023 |
| | | | US | 2020397313 A1 | 24-12-2020 |
| | | | US | 2023089991 A1 | 23-03-2023 |
| | | | WO | 2019070978 A1 | 11-04-2019 |
| US 2019269344 | A1 | 05-09-2019 | CA | 3104720 A1 | 12-09-2019 |
| | | | EP | 3787491 A1 | 10-03-2021 |
| | | | US | 2019269344 A1 | 05-09-2019 |
| | | | WO | 2019173399 A1 | 12-09-2019 |
| US 9042970 | B2 | 26-05-2015 | EP | 2582291 A1 | 24-04-2013 |
| | | | US | 2013085364 A1 | 04-04-2013 |
| | | | WO | 2011159250 A1 | 22-12-2011 |
| CA 2966180 | A1 | 06-05-2016 | AU | 2015338967 A1 | 11-05-2017 |
| | | | AU | 2020213276 A1 | 27-08-2020 |
| | | | AU | 2022200804 A1 | 24-02-2022 |
| | | | CA | 2966180 A1 | 06-05-2016 |
| | | | CN | 107205679 A | 26-09-2017 |
| | | | CN | 113057649 A | 02-07-2021 |
| | | | CN | 116530951 A | 04-08-2023 |
| | | | EP | 3212061 A1 | 06-09-2017 |
| | | | EP | 4218580 A1 | 02-08-2023 |
| | | | JP | 7022729 B2 | 18-02-2022 |
| | | | JP | 2018504148 A | 15-02-2018 |
| | | | JP | 2020022792 A | 13-02-2020 |
| | | | JP | 2022023914 A | 08-02-2022 |
| | | | KR | 20170075012 A | 30-06-2017 |
| | | | KR | 20200003284 A | 08-01-2020 |
| | | | KR | 20210148439 A | 07-12-2021 |
| | | | KR | 20220138418 A | 12-10-2022 |
| | | | US | 2016120433 A1 | 05-05-2016 |
| | | | US | 2016120434 A1 | 05-05-2016 |
| | | | US | 2017188872 A1 | 06-07-2017 |
| | | | US | 2018242876 A1 | 30-08-2018 |
| | | | US | 2019046066 A1 | 14-02-2019 |
| | | | US | 2019274574 A1 | 12-09-2019 |
| | | | US | 2020289014 A1 | 17-09-2020 |
| | | | US | 2021217519 A1 | 15-07-2021 |
| | | | US | 2022093247 A1 | 24-03-2022 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 23 18 0353

09-10-2023

| Patent document cited in search report | Publication date | Patent family member(s) | | Publication date |
|---|---|---|---|---|
| | | US | 2023207122 A1 | 29-06-2023 |
| | | WO | 2016070128 A1 | 06-05-2016 |

EPO FORM P0459